# FASCICULE DE BREVET EUROPEEN

(11) **EP 0 511 893 B1**
(45) Date de publication et mention de la délivrance du brevet: **14.12.1994**
(21) Numéro de dépôt: 92401086.1
(22) Date de dépôt: 17.04.1992
(51) Int. Cl.: B01J 19/10, A45D 33/00, A61K 7/00, B65B 1/24, B29C 67/22, B29B 15/10

(54) **Procédé de compactage sur un support d'un mélange pulvérulent et applicateur de maquillage constitué par un support portant une pastille de mélange pulvérulent compacté**
Verfahren zur Verdichtung eines Pulvergemischs auf einer Unterlage und Maskarastift bestehend aus einer Unterlage mit einer Pastille von kompaktiertem Pulvergemisch
Process for compacting a powder mixture on a support and mascara applicator comprising a support carrying a pad of compacted powder mixture

(30) Priorité: 29.04.1991 FR 9105244
(43) Date de publication de la demande: 04.11.1992
(73) Titulaire: L'OREAL, 75008 Paris (FR)
(72) Inventeur: Gueret, Jean-Louis, F-75018 Paris (FR)
(74) Mandataire: Peuscet, Jacques

(56) Documents cités:
- EP-A- 0 123 766
- EP-A- 0 310 472
- FR-A- 2 168 625
- US-A- 4 938 952
- PATENT ABSTRACTS OF JAPAN vol. 13, no. 95 (C-573)(3443) 6 Mars 1989
- PATENT ABSTRACTS OF JAPAN vol. 13, no. 414 (C-635)(3762) 13 Septembre 1989

## Description

La présente invention concerne un procédé de compactage sur un support d'un mélange pulvérulent et un applicateur de maquillage constitué par le support portant une pastille de mélange pulvérulent compacté obtenu par ce procédé.

Dans la demande de brevet français 9O O9327 déposée le 2O Juillet 199O par la demanderesse, on a décrit un procédé de compactage d'un mélange pulvérulent dans lequel on dépose une couche de mélange pulvérulent au fond d'une enceinte, on fait coulisser dans cette enceinte un piston, qui exerce directement une pression sur le mélange pulvérulent, et, pendant que l'on exerce cette pression, on soumet le mélange pulvérulent à l'action d'ultrasons ; selon la demande ci-dessus indiquée, le mélange pulvérulent contient de 5 à 8O % d'au moins un produit pulvérulent thermoplastique, le reste étant constitué par au moins un produit non thermoplastique. La présence d'un produit pulvérulent thermoplastique dans le mélange-à compacter permet sous l'action des ultrasons de créer dans le mélange pulvérulent une armature interne et/ou externe qui maintient le produit non thermoplastique.

La demanderesse a cherché à utiliser ce procédé pour préparer des applicateurs, plus particulièrement des applicateurs de faible dimension utilisés comme échantillons, en compactant sur un support le mélange pulvérulent. On a constaté que, si la présence de la substance thermoplastique permettait de fixer le mélange compacté au support, le délitage du mélange compacté n'était pas satisfaisant ; la seule méthode pour augmenter la quantité de poudre délitable était d'augmenter l'épaisseur de la couche de mélange compacté sur le support ; mais l'épaisseur nécessaire pour obtenir une fraction délitable satisfaisante donne alors à l'applicateur un aspect inesthétique et entraîne une perte de produit puisque la fraction délitable est faible par rapport à l'ensemble de la couche compactée.

Il était donc souhaitable de trouver un procédé permettant de fixer le mélange compacté sur un support sans qu'il soit nécessaire de mettre une épaisseur trop importante de poudre sur ledit support.

Selon la présente invention, on a trouvé que l'on peut résoudre ce problème en utilisant un support présentant, au moins sur la surface en contact avec le mélange pulvérulent, des anfractuosités (c'est-à-dire, notamment, des petites cavités, des pores, des cellules, des irrégularités, des poils ou des fibres) et en appliquant la pression et les ultrasons sur le mélange à compacter à travers le support.

La présente invention a, par conséquent, pour premier objet un procédé de compactage d'un mélange pulvérulent sur un support selon lequel on dépose une couche de mélange pulvérulent à compacter au fond d'une enceinte, on exerce une pression sur le mélange à compacter à l'aide d'un piston coulissant dans cette enceinte et, simultanément, on fait agir sur ledit mélange des ultrasons, caractérisé par le fait que l'on place, sur la couche de mélange pulvérulent, un support, dont au moins la surface en contact avec le mélange pulvérulent présente des anfractuosités, et que l'on exerce la pression et fait agir les ultrasons sur le mélange pulvérulent à travers le support.

On a constaté qu'avec ce procédé, en l'absence de produit thermoplastique, le mélange pulvérulent se fixait de façon satisfaisante sur le support et le mélange pulvérulent compacté se délitait convenablement.

Le mélange pulvérulent est constitué de tout produit minéral ou organique ou de tout mélange de ces produits susceptibles d'être compactés dans les conditions opératoires. Il est plus particulièrement constitue de produit(s) destiné(s) au maquillage, par exemple de la peau du visage ou des paupières. Parmi les produits minéraux, on peut citer le talc, les argiles, en particulier le kaolin, le mica et les pigments minéraux, par exemple les oxydes de titane, de zinc ou de fer. Parmi les produits organiques, on peut citer des poudres végétales, telles que l'amidon de riz ou la poudre de soie, des poudres de polymères non thermoplastiques, tels que les polyacrylates, et des fibres, par exemple des fibres de coton.

Il est possible d'introduire éventuellement dans le mélange à compacter une petite quantité de produit thermoplastique pour améliorer la cohésion du mélange après compactage. Cette quantité est, avantageusement, inférieure à 5 %.

Comme expliqué ci-dessus, par le terme "anfractuosités", on désigne de petites cavités, des pores, des cellules, des irrégularités, des aspérités ou des intervalles entre des poils ou des fibres. Ces anfractuosités sont obtenues en utilisant des supports en mousse de matière plastique à cellules ouvertes ou semi-ouvertes, des supports en mousse de cellulose, des supports en coton comprimé ou des supports en matière non-tissée. On peut également utiliser des supports recouverts, sur au moins une face, d'un flocage ou d'une couche de mousse ; dans ce cas, le support est, de préférence, en plastique non thermofusible rigide ou semi-rigide, ou en carton. On peut cependant, utiliser des mousses floquées ou des non-tissés floqués. De façon connue, le flocage est effectué en collant sur le support à l'aide d'un vernis, des fils ou poils en rayonne, en coton, en résine acrylique ou polyamide, par exemple.

Les anfractuosités ont, de préférence, des dimensions supérieures au diamètre des grains du mélange pulvérulent.

On peut penser qu'au cours du compactage, le mélange pulvérulent s'accroche dans certaines zones du support, car les ultrasons dispersent la poudre dans les anfractuosités constituées, par exemple, par les cellules des mousses ou les intervalles entre les fibres du flocage.

Les supports peuvent avoir des formes très variables telles que bandelettes, palettes circulaires ou ovales, munies ou non d'une tige ou queue permettant de saisir l'applicateur.

Selon une caractéristique du procédé de la présente invention, la pression et les ultrasons sont appliqués sur le mélange pulvérulent à travers le support. On obtient ainsi un compactage plus homogène que lorsque la pression et les ultrasons sont appliqués directement sur le mélange pulvérulent. D'autre part, la construction de l'enceinte de compactage est facilitée, ce qui permet de fabriquer des applicateurs de formes variées.

Les ultrasons utilisés selon l'invention ont avantageusement une fréquence comprise entre 1O et 1OO kHz et une amplitude comprise entre 2O et 6O µm, la puissance envoyée dans le mélange pulvérulent au cours du compactage étant comprise entre 1 et 3 kw par cm³ de mélange compacté. La durée d'émission des ultrasons est comprise entre O,25 et 3 s.

La pression exercée est, de préférence, comprise entre 6O x 10⁵ et 1OO x 10⁵ Pascals.

Le piston applique sur le support pour effectuer le compactage est avantageusement constitué par la sonotrode d'un générateur d'ultrasons.

L'enceinte se compose généralement de deux parties superposées : une partie supérieure située du côté de l'ouverture de l'enceinte ayant, vue en plan, la forme du support et une partie inférieure située du côté du fond de l'enceinte ayant, vue en plan, la forme que l'on désire donner à la pastille compactée de mélange pulvérulent. Le fond de l'enceinte peut être plan, concave ou convexe ; il peut également être muni de motifs décoratifs en relief ou en creux.

On peut disposer sur le fond de l'enceinte une couche de matériau élastomère, par exemple de silicone. On peut également disposer sur le fond de l'enceinte un grillage sur lequel on viendra mettre le mélange pulvérulent et qui servira d'évent au cours de la compression.

La face d'extrémité du piston peut être plane, concave ou convexe.

Selon un mode de mise en oeuvre particulier du procédé selon l'invention, le support est disposé avec son emballage sur le fond de l'enceinte de compactage, le mélange pulvérulent à compacter étant mis en place entre ledit emballage et ledit support, l'emballage ayant une forme telle que le piston puisse appuyer sur la partie de la face du support opposée à celle en contact avec le mélange pulvérulent à compacter.

La présente invention a pour second objet un applicateur obtenu par le procédé selon l'invention constitué par un support portant au moins une pastille de mélange pulvérulent compacté.

Selon l'invention, le support peut porter une ou plusieurs pastilles ; dans ce dernier cas, l'applicateur peut constituer une palette de produits compactés de diverses couleurs.

Selon l'invention, le support présente avantageusement des anfractuosités sur toute sa surface externe, la pastille de mélange pulvérulent compacté étant disposée au droit d'une partie desdites anfractuosités. Par exemple, le support est entièrement constitué par une mousse, ou entièrement constituée par du coton comprimé, ou constitué par un support entièrement floqué, ou encore entièrement recouvert de mousse ; dans tous ces cas, on peut utiliser une face de l'applicateur pour l'application de la poudre de maquillage et l'autre face pour estomper le maquillage appliqué.

L'applicateur selon l'invention est, de préférence, protégé par un emballage. L'applicateur peut être introduit dans l'emballage après ou avant le compactage du mélange pulvérulent. L'emballage peut être constitué par une coque en matière thermoplastique injectée ou formée ou un complexe de carton compact découpé contenant l'applicateur et fermé sur ses deux faces par deux opercules, éventuellement transparents, dont l'un au moins est pelable.

La description donnée ci-dessous, à titre illustratif et non limitatif, d'un mode de réalisation de l'invention représenté sur le dessin annexé, permettra de mieux comprendre l'invention.

Sur ce dessin :
- la figure 1 représente, en perspective, un applicateur selon l'invention ;
- la figure 2 représente, en section verticale, l'enceinte de compactage avant compactage ;
- la figure 3 représente la même enceinte après compactage ;
- la figure 4 représente l'applicateur de la figure 1 dans un emballage de protection.

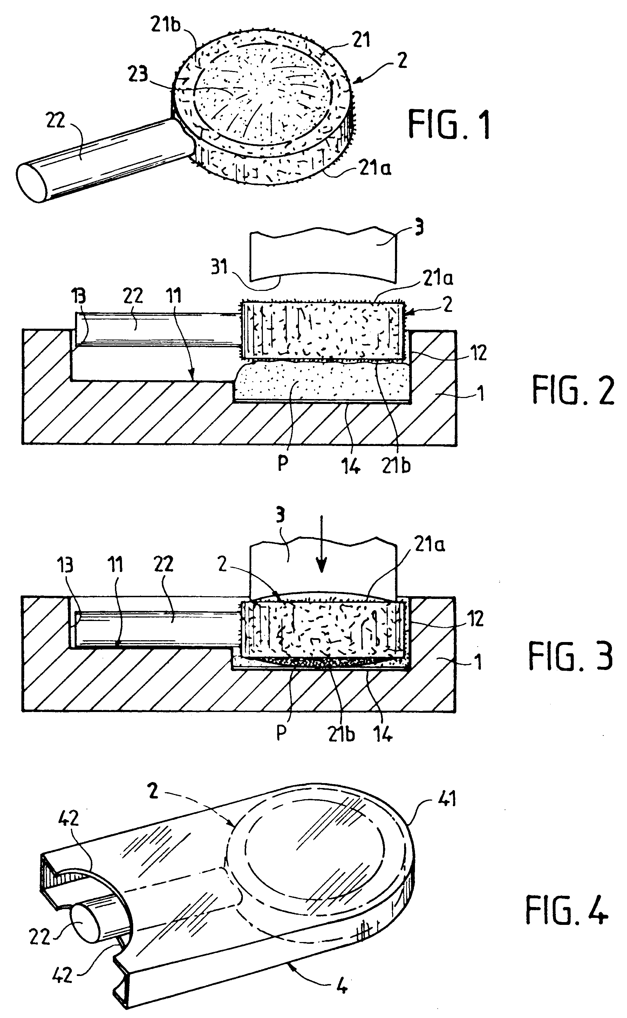

Comme illustré sur les figures 2 et 3 pour effectuer le compactage, on introduit, dans un moule 1, une couche de mélange pulvérulent P et un support 2; on exerce ensuite une pression sur le mélange pulvérulent P à travers le support 2 à l'aide d'un piston 3 constitué par la sonotrode d'un générateur d'ultrasons.

Dans le mode de réalisation représenté, le support a la forme d'une palette 21 en forme de disque munie d'une queue 22 permettant de saisir le support. La palette 21 a un diamètre de 8 mm et une épaisseur de 4 mm ; elle est entièrement floquée, c'est-à-dire sur ses deux faces, à l'aide d'un floc de fibres de rayonne ayant 1 mm de long et O,O6 mm de diamètre.

Dans le moule 1 est ménagée une enceinte 11 ouverte vers le haut qui est divisée en deux parties 12 et 13 ; la partie inférieure 12 située du côte du fond 14 a la forme et les dimensions, au jeu nécessaire près, de la palette 21, la partie supérieure 13 a la forme de l'ensemble du support 2, c'est-à-dire de la palette 21 et de la queue 22. L'enceinte a dans la partie 12 un diamètre de 1O mm et, dans sa partie la plus profonde, une profondeur de 4 mm. Le fond 14 de l'enceinte est plan.

Pour effectuer le compactage, on introduit sur le fond 14 de l'enceinte O,2 ml de poudre pigmentaire non thermoplastique contenant 4 % de stéarate de magnésium comme liant. On abaisse ensuite la sonotrode 3 jusqu'à ce qu'elle soit en contact avec la face supérieure 21a du support 2 ; on applique grâce à la sonotrode sur ledit support 2 une pression de 60 x10⁵ Pascals pendant 2,5 s. La sonotrode, pendant la compression émet des ultrasons pendant 2 s, la puissance envoyée sous forme d'ultrasons au cours du compactage étant de 2 kw/cm³. L'extrémité 31 de la sonotrode 3 appuyant sur le support 2 est concave. On obtient ainsi une pastille 23 de mélange pulvérulent qui est fixée sur la face inférieure 21b de la palette 21. La surface 31 de la sonotrode 3 étant concave, la cohésion de la poudre est plus grande à la périphérie de la pastille 23 qu'au centre. On a mesuré que 9O % en poids du mélange pulvérulent compacté formant la pastille 23 sont délitables.

Pour protéger l'applicateur, on l'introduit dans un emballage 4 constitué par une boîte ayant sensiblement une forme de parallélépipède rectangle dont l'un des petits côtés est ouvert et dont l'autre petit côté constitue un demi cylindre 41 ayant, au jeu nécessaire près, le même diamètre que la palette 21. La longueur totale de la boîte est légèrement supérieure à la plus grande dimension de l'applicateur 2, c'est-à-dire au total de la longueur de la queue 22 et du diamètre de la palette 21. L'épaisseur de la boîte est égale, au jeu nécessaire près, à l'épaisseur de la palette 21 y compris l'épaisseur de la pastille 23 compactée de mélange pulvérulent. Les grandes faces du parallélépipède sont munies du côté ouvert d'encoches 42 permettant à l'utilisateur de saisir la queue 22 pour sortir l'applicateur 2.

Lorsque l'utilisateur désire utiliser l'applicateur 2 pour se maquiller à l'aide du mélange pulvérulent, il le sort de l'emballage 4 en le saisissant par la queue 22. Il applique ensuite sur la peau ou les paupières le mélange pulvérulent en frottant sur la peau la face 21b de l'applicateur portant la pastille 23 pour provoquer son délitage ; ensuite, pour estomper le maquillage, il utilise la face 21a de l'applicateur. Il jette l'applicateur après cet usage unique.

## Revendications

1. Procédé de compactage sur un support (2) d'un mélange pulvérulent (P), dans lequel on dépose une couche de mélange pulvérulent à compacter au fond d'une enceinte (11), on fait coulisser dans l'enceinte un piston (3), on exerce une pression sur le mélange à compacter à l'aide d'un piston coulissant dans cette enceinte et, simultanément, on fait agir sur ledit mélange des ultrasons, caractérisé par le fait que l'on place sur la couche de mélange pulvérulent à compacter un support (2), dont au moins la surface (21b) en contact avec le mélange pulvérulent présente des anfractuosités, et que l'on exerce la pression en faisant agir les ultrasons sur le mélange à compacter à travers le support (2).

2. Procédé selon la revendication 1, caractérisé par le fait que le mélange à compacter contient une poudre thermoplastique en quantité inférieure à 5 %.

3. Procédé selon l'une des revendications 1 ou 2, caractérisé par le fait que l'on utilise un support (2) en mousse de matière plastique à cellules ouvertes ou semi-ouvertes, un support en mousse de cellulose, un support en coton comprimé ou un support en non-tissé.

4. Procédé selon l'une des revendications 1 ou 2, caractérisé par le fait qu'on utilise soit un support (2) rigide ou semi-rigide en matériau plastique non thermofusible ou en carton, au moins partiellement floqué ou recouvert d'une couche de mousse, soit un support en mousse en non-tissé floqué.

5. Procédé selon l'une des revendications 1 à 4, caractérisé par le fait que les anfractuosités ont des dimensions supérieures au diamètre des particules du mélange pulvérulent.

6. Procédé selon l'une des revendications 1 à 5, caractérisé par le fait que les ultrasons ont une fréquence comprise entre 1O et 1OO kHz et une amplitude comprise entre 2O et 6O µm, la puissance envoyée dans le mélange pulvérulent au cours du compactage étant comprise entre 1 et 3 kw par cm³ de mélange compacté.

7. Procédé selon l'une des revendications 1 à 6, caractérisé par le fait que la durée d'émission des ultrasons est comprise entre O,25 et 3 s.

8. Procédé selon l'une des revendications 1 à 7, caractérisé par le fait que la pression exercée pendant l'emission d'ultrasons est comprise entre 60 x 10⁵ et 100 x 10⁵ Pascals.

9. Procédé selon l'une des revendications 1 à 8, caractérisé par le fait que le piston appliqué sur le support pour effectuer le compactage est constitué par la sonotrode d'un générateur d'ultrasons.

10. Procédé selon l'une des revendications 1 à 9, caractérisé par le fait que l'on dispose sur le fond de l'enceinte une couche de matériau élastomère.

11. Procédé selon l'une des revendication 1 à 10, caractérisé par le fait qu'on dispose sur le fond de l'enceinte une grille.

12. Procédé selon l'une des revendications 1 à 11 caractérisé par le fait que l'on dispose le support avec son emballage dans le fond (14) de l'enceinte (11) de compactage, le mélange pulvérulent à compacter étant mis en place entre ledit emballage et ledit support et l'emballage ayant une forme telle que le piston puisse appuyer sur la partie de la face du support opposée à celle en contact avec le mélange pulvérulent à compacter.

13. Applicateur susceptible d'être obtenu par le procédé selon l'une des revendications 1 à 12, constitué par un support portant au moins une pastille de mélange pulvérulent compacté.

14. Applicateur selon la revendication 13, caractérisé par le fait que le support présente des anfractuosités sur toute sa surface externe, la pastille étant disposée au droit d'une partie au moins desdites anfractuosités.

15. Applicateur selon l'une des revendications 13 ou 14, caractérisé par le fait qu'il est protégé par un emballage.

## Claims

1. Process for compacting a powder mixture (P) on a support (2), in which a layer of powder mixture to be compacted is deposited at the bottom of a chamber (11); a piston (3) is made to slide in the chamber; a pressure is exerted on the mixture to be compacted with the aid of a piston sliding in this chamber; and, simultaneously, ultrasonic waves are made to act on the said mixture; characterized in that a support (2) is placed on the layer of powder mixture to be compacted, at least the surface (21b) of which support in contact with the powder mixture, exhibits anfractuosities; and in that the pressure is exerted by making the ultrasonic waves act on the mixture to be compacted through the support (2).

2. Process according to Claim 1 characterized in that the mixture to be compacted contains a thermoplastic powder in a quantity of less than 5%.

3. Process according to one of Claims 1 or 2, characterized in that a support (2) made of open-cell or semi-open-cell plastic foam, a support made of cellulose foam, a support made of compressed cotton, or a support made of a nonwoven is used.

4. Process according to one of Claims 1 or 2, characterized in that either a rigid or semi-rigid support (2) made of non-thermofusible plastic or of board, at least partially flocked or covered with a layer of foam, or a support made of foam or a flocked nonwoven, is used.

5. Process according to one of Claims 1 to 4, characterized in that the anfractuosities have dimensions greater than the diameter of the grains of the powder mixture.

6. Process according to one of Claims 1 to 5, characterized in that the ultrasonic waves have a frequency of between 10 and 100 kHz and an amplitude of between 20 and 60 µm, the power delivered into the powder mixture in the course of compacting being between 1 and 3 kw per cm³ of compacted mixture.

7. Process according to one of Claims 1 to 6, characterized in that the emission time of the ultrasonic waves is between 0.25 and 3 s.

8. Process according to one of Claims 1 to 7, characterized in that the pressure exerted during the ultrasonic emission is between 60 × 10⁵ and 100 × 10⁵ pascals.

9. Process according to one of Claims 1 to 8, characterized in that the piston applied to the support in order to effect compacting is constituted by the sonotrode of an ultrasonic generator.

10. Process according to one of Claims 1 to 9, characterized in that a layer of elastomeric material is arranged on the bottom of the chamber.

11. Process according to one of Claims 1 to 10, characterized in that a grating is arranged on the bottom of the chamber.

12. Process according to one of Claims 1 to 11, characterized in that the support is arranged with its package on the bottom (14) of the compacting chamber (11), the powder mixture to be compacted being put into place between the said package and the said support, and the package having a shape such that the piston can press on that part of the face of the support opposite that in contact with the powder mixture to be compacted.

13. Applicator capable of being obtained by the process according to one of Claims 1 to 12, constituted by a support carrying at least one pad of compacted powder mixture.

14. Applicator according to Claim 13, characterized in that the support exhibits anfractuosities over its entire external surface, the pad being arranged in line with at least part of the said anfractuosities.

15. Applicator according to one of Claims 13 and 14, characterized in that it is protected by a package.

## Patentansprüche

1. Verfahren zum Kompaktieren einer pulverförmigen Mischung (P) auf einem Halter (2), wobei man eine Schicht der zu kompaktierenden pulverförmigen Mischung auf den Boden einer Fassung (11) aufträgt, einen Stempel (3) in die Fassung einführt, mit Hilfe des in der Fassung beweglichen Stempels einen Druck auf die zu kompaktierende Mischung ausübt und wobei man gleichzeitig Ultraschall auf die Mischung einwirken läßt,
**dadurch gekennzeichnet**, daß
man einen Halter (2) auf die Schicht der zu kompaktierenden pulverförmigen Mischung legt, von dem wenigstens die mit der pulverförmigen Mischung in Kontakt stehende Fläche (21b) Vertiefungen aufweist, und daß man den Druck ausübt, wobei der Ultraschall durch den Halter (2) hindurch auf die zu kompaktierende Mischung einwirkt.

2. Verfahren gemäß Anspruch 1, dadurch gekennzeichnet, daß die zu kompaktierende Mischung weniger als 5 % eines thermoplastischen Pulvers aufweist.

3. Verfahren gemäß einem der Ansprüche 1 oder 2, dadurch gekennzeichnet, daß man einen Halter (2) aus einem Schaumstoff auf Kunststoffbasis mit offenen oder halboffenen Zellen verwendet, einen Halter aus einem Celluloseschaumstoff, einen Halter aus gepreßter Baumwolle oder einen Halter aus einem Faservlies.

4. Verfahren gemäß einem der Ansprüche 1 oder 2, dadurch gekennzeichnet, daß man entweder einen starren oder halbstarren Halter (2) aus einem wärmefesten Kunststoff oder aus Karton, der wenigstens teilweise beflockt oder mit einer Schaumstoffschicht bedeckt ist, oder einen Halter aus einem Schaumstoff oder einem beflockten Faservlies verwendet.

5. Verfahren gemäß einem der Ansprüche 1 bis 4, dadurch gekennzeichnet, daß die Vertiefungen größer dimensioniert sind als der Durchmesser der Teilchen der pulverförmigen Mischung.

6. Verfahren gemäß einem der Ansprüche 1 bis 5, dadurch gekennzeichnet, daß der Ultraschall eine Frequenz zwischen 10 und 100 kHz und eine Amplitude zwischen 20 und 60 µm aufweist, wobei die der pulverförmigen Mischung während der Kompaktierung zugeführte Leistung zwischen 1 und 3 kW pro cm³ der kompaktierten Mischung beträgt.

7. Verfahren gemäß einem der Ansprüche 1 bis 6, dadurch gekennzeichnet, daß die Dauer der Ultraschallemission zwischen 0,25 und 3 s beträgt.

8. Verfahren gemäß einem der Ansprüche 1 bis 7, dadurch gekennzeichnet, daß der während der Ultraschallemission ausgeübte Druck zwischen 60 x 10⁵ und 100 x 10⁵ Pascal beträgt.

9. Verfahren gemäß einem der Ansprüche 1 bis 8, dadurch gekennzeichnet, daß der zur Kompaktierung auf den Halter einwirkende Stempel von dem Schallkopf eines Ultraschallgenerators gebildet wird.

10. Verfahren gemäß einem der Ansprüche 1 bis 9, dadurch gekennzeichnet, daß man auf den Boden der Fassung eine Schicht eines Elastomers aufträgt.

11. Verfahren gemäß einem der Ansprüche 1 bis 10, dadurch gekennzeichnet, daß man auf dem Boden der Fassung ein Gitter anordnet.

12. Verfahren gemäß einem der Ansprüche 1 bis 11, dadurch gekennzeichnet, daß man den Halter samt seiner Hülle auf dem Boden (14) der zur Kompaktierung dienenden Fassung (11) anordnet, wobei sich die zu kompaktierende, pulverförmige Mischung zwischen der Hülle und dem Halter befindet und wobei die Hülle so geformt ist, daß der Stempel auf dem Teil der Seite des Halters aufliegen kann, der demjenigen, welcher in Kontakt mit der zu kompaktierenden pulverförmigen Mischung steht, gegenüberliegt.

13. Applikator, erhältlich nach dem Verfahren gemäß einem der Ansprüche 1 bis 12, bestehend aus einem Halter, der wenigstens eine Pastille einer kompaktierten, pulverförmigen Mischung aufweist.

14. Applikator gemäß Anspruch 13, dadurch gekennzeichnet, daß der Halter auf seiner gesamten Außenseite Vertiefungen aufweist, wobei die Pastille auf wenigstens einem Teil dieser Vertiefungen angeordnet ist.

15. Applikator gemäß einem der Ansprüche 13 oder 14, dadurch gekennzeichnet, daß er von einer Hülle geschützt ist.
